Europäisches Patentamt

European Patent Office

Office européen des brevets

(11) Publication number: **0 281 380 B1**

# EUROPEAN PATENT SPECIFICATION

(45) Date of publication of patent specification: **29.11.95**  (51) Int. Cl.⁶: **A61K  38/20**, A61K 31/70

(21) Application number: **88301821.0**

(22) Date of filing: **02.03.88**

(54) **Synergistic interplay of interleukin-2 and dsRNAs.**

(30) Priority: **03.03.87 US 21368**

(43) Date of publication of application:
**07.09.88 Bulletin  88/36**

(45) Publication of the grant of the patent:
**29.11.95 Bulletin  95/48**

(84) Designated Contracting States:
**AT BE CH DE ES FR GB GR IT LI LU NL SE**

(56) References cited:
**EP-A- 0 113 162**
**EP-A- 0 213 921**

**JOURNAL OF BIOLOGICAL RESPONSE MODI-
FIERS, vol. 6, no. 5, 1987, Raven Press Ltd.,
New York, NY (US); H.R. HUBBELL et al., pp.
525-536/**

**DIALOG, no. 6160495, embase no. 86155555;
M.S. CHAPEKAR et al./**

**DIALOG, no. 6109766, embase no. 86104826;
H.R. HUBBELL et al./**

**JOURNAL OF UROLOGY, vol. 131, 1984; pp.
223-226/**

**CANCER IMMUNOLOGY & IMMUNOTHE-
RAPHY, vol. 34, 1992; pp. 259-264/**

**CANCER IMMUNOLOGY & IMMUNOTHE-
RAPHY, vol. 35, 1992; pp. 151-157/**

(73) Proprietor: **HEM PHARMACEUTICALS CORP.
12280 Wilkins Avenue
Rockville, MD 20852 (US)**

(72) Inventor: **Carter, William A.
1 Jaine Lane
Birchrunville, PA (US)**

(74) Representative: **Votier, Sidney David et al
CARPMAELS & RANSFORD
43, Bloomsbury Square
London WC1A 2RA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Present use of lymphokines (interleukin-1, 2 and 3, interferon, tumor necrosis factor, etc.) is handicapped severely because of narrow therapeutic range and severe toxicity usually observed at the high doses necessary to effect responses in human cancers or viral diseases. The present invention describes unexpected synergistic interplay between IL-2 and dsRNAs which dramatically reduce the necessary dosages of IL-2,and simultaneously widen their therapeutic range. Specific combinations of dsRNA plus IL-2 in cancer, viral and inflammatory disease treatments significantly increase survival in human melanoma-bearing animals in part because of augmentation by dsRNA of lymphokine activated killer cells (LAK) which are first formed in response to IL-2 exposure. The invention has wide applicability to non-toxic, efficacious control of both human cancer and various human viral infections.

## BACKGROUND OF THE INVENTION

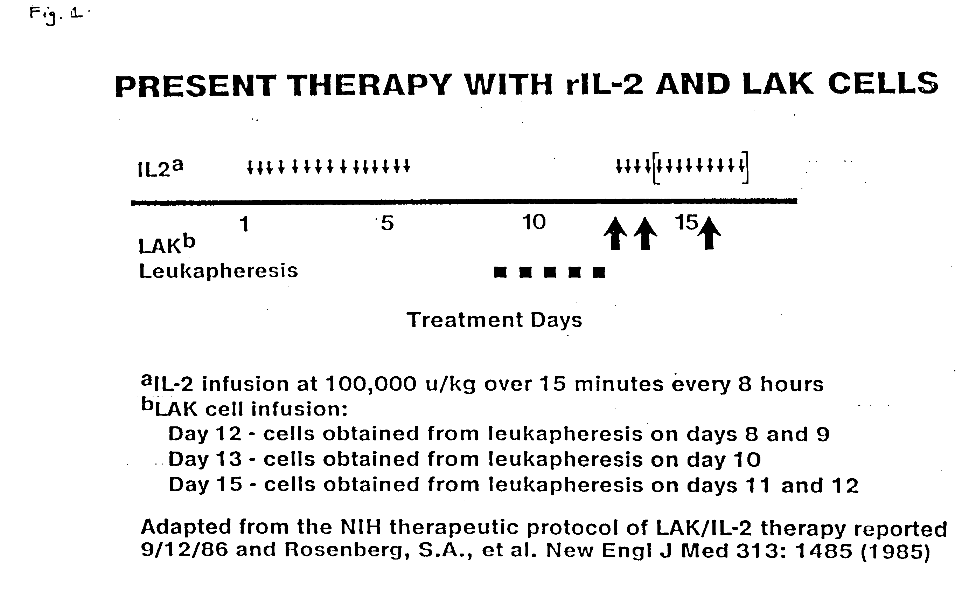

**Present Therapy with Interleukin-2 (IL-2) as the Prototype Lymphokine.** Lymphokines are now routinely manufactured by recombinant DNA technology. rIL-2 refers to recombinant interleukin-2. LAK refers to lymphokine activated killer cells. The latter cells are generated in vitro by exposing human blood cells (obtained by leukapheresis) to rIL-2. The cells are then customarily reinfused into the patient where it is at least hoped that the LAK cells, with continued IL-2 stimulation, will kill or control the patient's tumor. The presently available regime is described in Figure 1. The arrows inside the brackets indicate IL-2 doses which usually cannot be given due to overwhelming toxicities. Toxicities reported in a clinical trial and measures used to counteract these toxic reactions are given in Tables 1 and 2. Due to the massive side-effects, therapeutic effects are only seen in 10-20% of patients treated with lymphokines (see Rosenberg et al New England Journal of Medicine, volume 313, p. 1485, 1985). The most responsive tumors seem to be kidney and melanoma when lymphokines are given by themselves, but my invention has wide applicability to other cancers, viral disorders in general and various inflammatory states. There is no evidence that their efficacy can be increased by combining them with routinely-used chemotherapeutic agents.

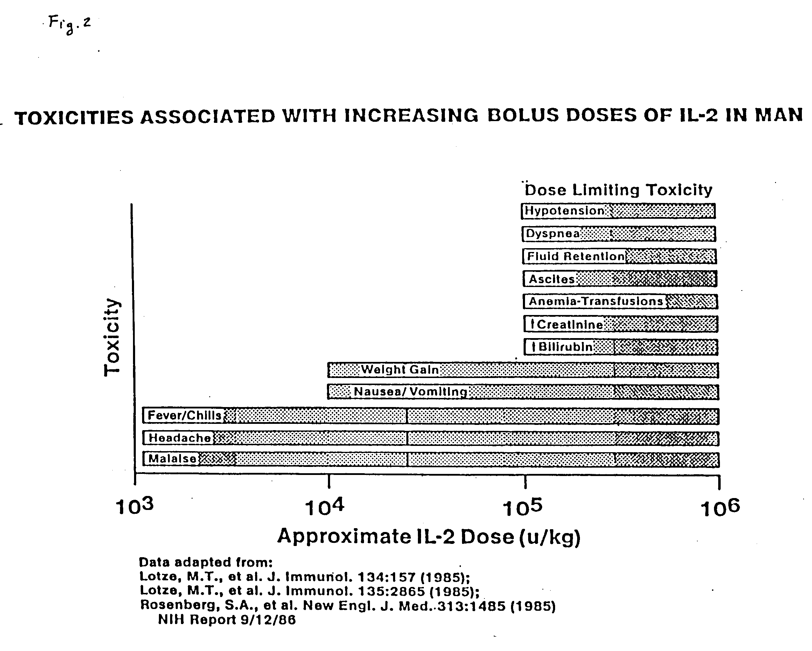

**Toxicities of Lymphokines are Dose-Related.** Figure 2 summarizes available clinical data which shows that at IL-2 doses greater than $10^5$ units per kilogram body weight, devastating toxicities can be seen in man. However, such doses have been generally deemed necessary in order to effect tumor regressions. Unfortunately, often the patient will die from the IL-2 toxicity before the tumor will become measurably smaller as lymphokine therapy is currently practiced.

EP-A-0 113 162 discloses therapeutic compositions of (mismatched) dsRNA and interferons (IFN), the combination acting synergistically to inhibit tumour cell proliferation, convert tumour cells to normal cells and to correct cancer-related immune defects. The synergistic effect furthermore allows the dosage of toxic IFN to be reduced. Synergistic cytocidal and antiproliferative effects of combinations of dsRNAs and IFNs on various tumour cell lines are also disclosed in Cancer Res 46 (1986) 1698 - 1702 and Int J Cancer 37 - (1986) 359 - 365. EP-A-0 213 921, which forms part of the piror art by virtue of the provisions of Article 54-(3) and (4) EPC, discloses the use of a dsRNA, optionally in combination with a lymphokine such as an interferon or an interleukin, in the treatment of retrovirus or T-cell lymphotropic virus induced conditions.

## BRIEF DESCRIPTION OF THE DRAWINGS

FIGURE 1 is a representation of current tumor therapy with recombinant Interleukin-2 (rIL-2) and lymphokine activated killer (LAK) cells and is adapted from the NIH therapeutic protocol of LAK/IL-2 therapy reported by Rosenberg, S.A. et al in New England Journal of Medicine 313:1485 (September 12, 1985);

FIGURE 2 is a bar graph summarizing available clinical data comparing various IL-2 doses with toxicity and dose limiting toxicity;

FIGURES 3A and 3B are bar graphs comparing the cytotoxicity at different doses of IL-2 with a constant dose (50:1 and 25:1, respectively) of dsRNA;

FIGURES 4A and 4B are bar graphs showing the increased human LAK cell activity of combination rIL-2 and dsRNA therapy;

FIGURE 5 is a bar graph reporting the number of melanoma lung metastases for four different groups including the combination of dsRNA and rIL-2;

FIGURE 6 is a graph comparing the survival in malignant melanoma xenograft bearing athymic mice for the indicated number of days for four different groups; and

FIGURE 7 is a bar graph similar to Figure 1, illustrating toxicities associated with increasing bolus doses of IL-2 in man and comparing current IL-2 dosage ranges with combined IL-2 and dsRNA projected doses.

## SUMMARY OF THE INVENTION

A synergistic combination of dsRNA, as the non-toxic member, and IL-2, as a toxic member, dramatically reduces the necessary doses of IL-2 to a moderate to no toxicity dose, and simultaneously widens their therapeutic range. The synergistic combination of IL-2 is effective to control human cancer, especially cancer of the lung and kidney and malignant melanoma or other disorders, according to the therapeutic methods described. The combination is also effective for treating various human viral infections. Therapeutic compositions are also described.

The methods and compositions of this invention present a dsRNA and IL-2 administered simultaneously or sequentially, which concentrations result in enhanced levels of both NK and LAK cells in the organism without increased toxicity, that is in excess of the toxicity, if any, of the individual component administered individually, resulting in an enhanced antitumor, antiviral or immunomodulatory response. Concurrently with this dsRNA/IL-2 therapy, my invention also includes the administration of a substance which inhibits cyclooxygenase activator. As an example, the invention includes concurrent administration of indomethacin, a prostaglandin inhibitor, thereby abrogating prostaglandin release and the resultant NK cell down regulation response.

In this manner, the natural inhibitors of desirable NK cells are removed, and the NK cell population increased accordingly. This invention includes the novel combinations -- therapeutic treatments and pharmaceutical compositions, where appropriate -- of dsRNA, lymphokine and a substance which inhibits cyclooxygenase activator.

Amounts of the dsRNA component provide a peak blood concentration of from 0.1 to 1000 micrograms dsRNA per milliliter in the systemic blood circulation immediately following administration measured at a point distal from the site of infusion. The amount of IL-2, preferably rIL-2, administered lies within a range of about $10^2$ IL-2 units per kg of the patient's body weight up to a value approaching unacceptable levels of toxicity, which may be as high as $10^6$ units. Most effective, toxic-reaction manageable values are in the range of from about $10^3$ to about $10^4$ IL-2 units per kg body weight.

*In vitro* and *in vivo* experiments confirm that dsRNAs (and mismatched dsRNAs in particular) confer a synergistic pharmacologic action with IL-2 which results in an enhanced biologic response (antitumor, antivirus, immune modulator) with greatly reduced toxicity. Typically, experiments with mismatched dsRNA demonstrate that the dosage of IL-2 required, as for an antitumor response, can be reduced at least 30 to 100 fold. The synergistic pharmacologic effect is conferred at (but not limited to) both the levels of NK (natural killer)-sensitive and also NK-resistant tumor cells. Cytotoxic cells with activity against NK-resistant tumor or viral-infected cells are also referred to as LAK (lymphokine activated killer) cells. I demonstrated this activity unequivocally by the use of monoclonal antibody markers in which I observed dramatic increases concurrently in both NK cells (using asialo-$G_{ml}$ monoclonal antibody) and LAK cells (using Thy 1.2 monoclonal antibody). Such antibodies are readily available monoclonal antibody reagents to those familiar with this art. I also observed that the concurrent use of compounds which reduce the biologic level of various NK cell natural antagonists (such as prostaglandins) would further amplify this synergy in an essentially non-toxic, or minimally toxic, manner. As an example, the use of indomethacin (25 mg per day for a 60 kilogram individual) dramatically increased the pharmacologic synergy in part by reducing endogenous prostaglandin concentration.

## DETAILED DESCRIPTION OF THE INVENTION

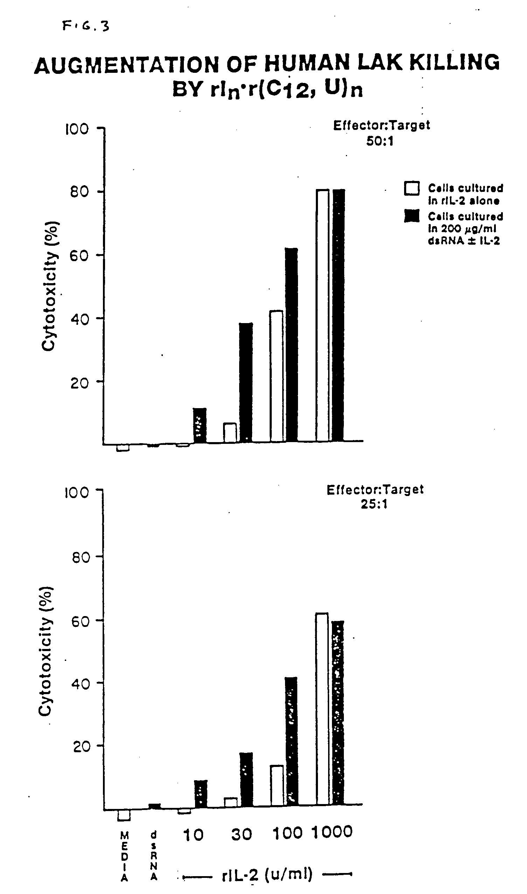

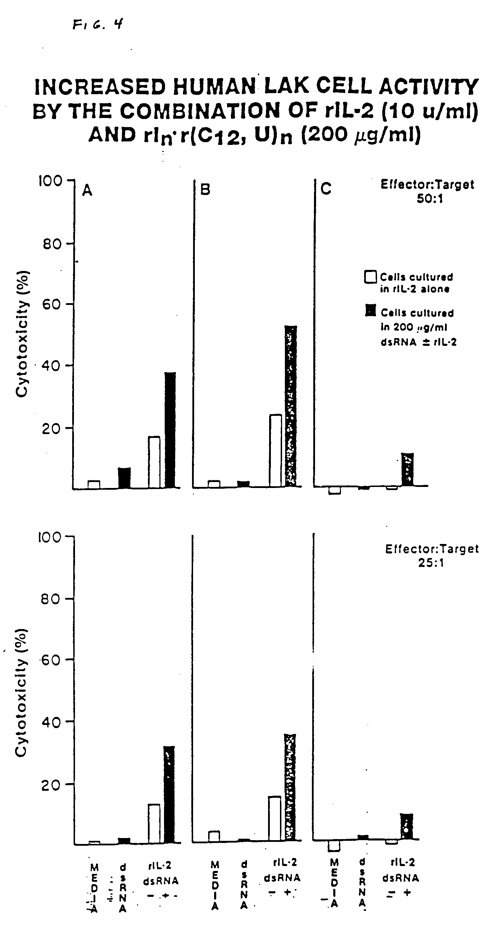

I have determined that dsRNAs act as biochemical catalysts to increase the specific killing of tumor cells by IL-2 generated LAK cells in a non-toxic manner. This is demonstrated by the following cell culture experiments. The human renal cancer cell line designated 7860 was grown in standard petri dishes and exposed to various potential killer cells. The peripheral blood cells obtained from normal individuals were designated "effector cells" (killer cells) and the renal cancer cells designated "target cells". The killing (termed cytotoxicity) was measured in various ways including use of supravital stains or radioisotopes: in general, dead tumor cells release previously engulfed radioactive metabolic precursors and can no longer take up extracellular nutrients, etc. Figure 3 shows that Ampligen, a mismatched dsRNA, significantly increases the activity of human LAK cells (obtained from blood of normal individuals) over that possible with IL-2 alone. Figure 3 compares the cytotoxicity at different doses of IL-2 with a constant dose of dsRNA. (At 1000 units per ml of IL-2, maximum cell killing has already occurred and it is not possible to boost it further with dsRNA.) Notice that the catalyst, dsRNA, in this system when used alone is completely inert. Therefore, the effect on tumor cell killing is not one of the simple arithmetic sum of 2 marginally active agents. Similar results are obtained in Figure 4, confirming the reproducibility of this remarkable phenomenon, which is equally applicable to a cell expressing neoantigens by virtue of either viral infection or a derangement in the self-immune (auto immune) regulation.

In separate experiments, it has also been shown that dsRNA (mismatched dsRNA) does not induce lymphocytes to produce IL-2. IL-2 concentration of in culture media was measured by the CTLL assay; the concentrations reported are expressed as the mean ± SEM. Rather, dsRNA may work in the IL-2 lymphokine system by various other means, including altering the density of certain antigens on target cells so that the efficiency of killing is increased. Also, dsRNA and IL-2 together may alter the late effects of killing, such as increasing effector-target cell binding or increasing the production of various cytotoxic factors or increasing the cycling of killer cells from one target tumor cell to the next target tumor cell.

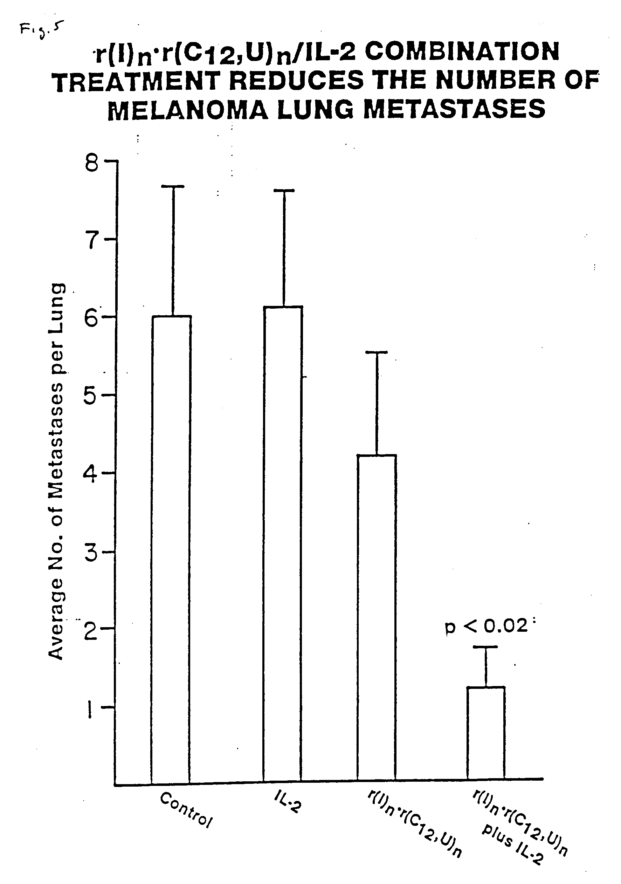

**Animal Studies show Increased Survival and Reduced Tumor Spread.** Increased survival and reduced tumor spread are shown by animal studies with the combination lymphokine/dsRNA over that with either the lymphokine or the dsRNA working alone. The human melanoma tumor designated "BRO" was injected into the foot pad of groups of athymic mice. Athymic mice are unable to reject as foreign a human tumor. Eventually, in untreated mice, the tumor cells proliferate, spread to the lungs, and the animals die. In Figure 5, treated mice received dosages of $10^3$-$10^4$ units/kilogram of IL-2 (about 2500 units per mouse) and dsRNA (500 micrograms) equivalent to about 100-300 mg for a 60 kilogram individual, both biologicals given twice weekly. The objective was to simulate the safe human dose of each biologic and determine if a safe dose was indeed also efficacious.

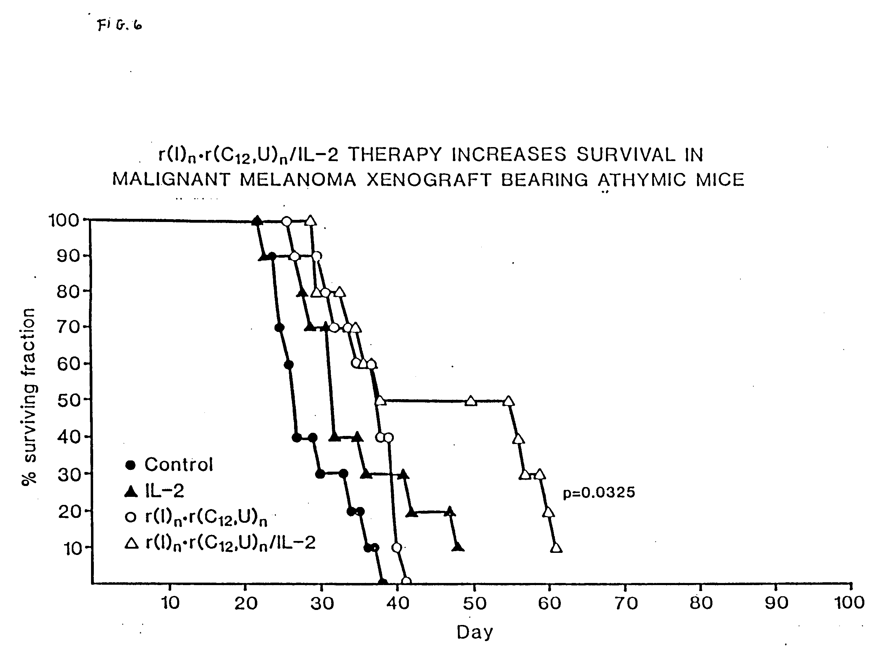

The data presented in Figure 5 show a dramatic reduction in metastases to the lungs of animals treated with dsRNA plus IL-2 which was obviously unexpected over the results with the two agents given singly. This reduction in lung metastases was also correlated with an increase in survival; see the data of Figure 6. By increasing the dose of the non-toxic member of the therapeutic combination - mismatched dsRNA - it should now be possible to increase survival indefinitely. Similar results have been obtained with interferon (alpha) 20,000 IRU wherein mismatched dsRNA (500 micrograms twice weekly) in combination resulted in further enhancement of tumor inhibition (p<0.02). Importantly, the lymphokine/dsRNA combinations did not produce detectable toxicity in the animals. They fed normally and showed normal blood chemistries.

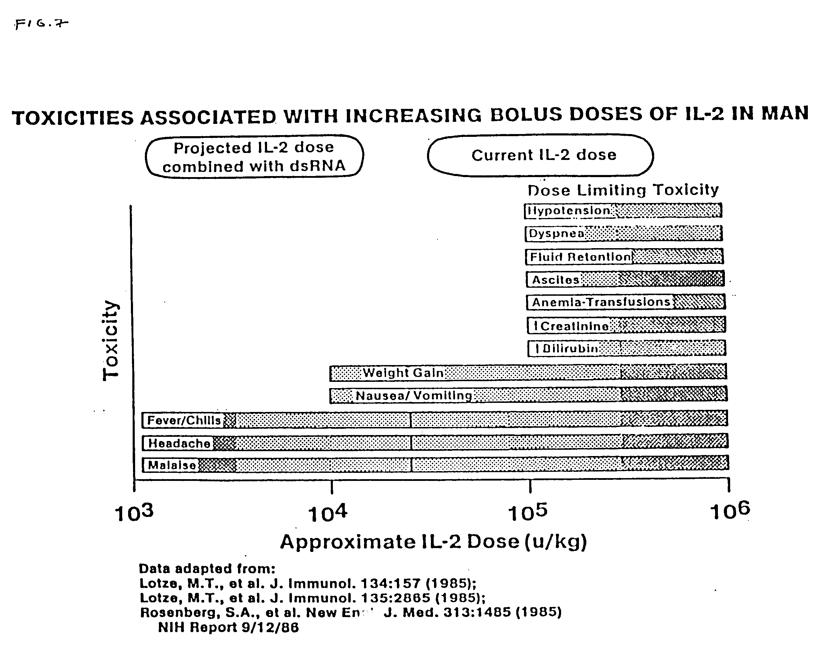

**Benefit of the Therapeutic Synergy at the Human Level.** Figure 7 shows how the combination therapy of the present invention will reduce the toxicity of lymphokines in general and IL-2 in particular in clinical medicine. The toxicity data of IL-2 are well established by Lotze and Rosenberg and present results indicate that IL-2 will now be effective in the usually low range of $10^3$-$10^4$ units per kilogram only if combined with dsRNAs, whereas, if not combined with dsRNAs, it will be largely ineffective at this safe dosage.

In viral disorders, similar therapeutic benefits will be obtained, both for the reasons already cited and additional ones as well. For example, therapeutic interventions which attack only the immune deficits (IL-2 responsive) in HIV infections appear to be counterproductive. Consider that the so-called T4 helper cells with TAC receptors are responsive to the lymphokine IL-2 but, in AIDS victims, IL-2 causes expansion of cells which are directly vulnerable to the HIV and, as a result, there is a worsening of the disease (A.S. Fauci and H.C. Lane, Annals Institute Pasteur/Virology-Immunology, in press, 1987). Thus, by judicious addition of dsRNA to IL-2, the spectrum of IL-2, and potentially other lymphokines such as the interferon and TNF, as useful antivirals should similarly enlarge and improve.

Mismatched double-stranded RNA is a known form of macromolecular RNA (see U.S. Patent No. 4,024,222 and U.S. Patent No. 4,130,641) in which destabilization of the double helix prevents base pairing. Mismatched dsRNA is well known for its interferon-induction properties which indicate a mechanism unrelated to interferon induction per se.

A typical therapeutic embodiment of mismatched double-stranded RNA is the synthetic dsRNA formed by complexes of polyriboinosinic and polyribocytidylic/uridylic acid, such as $rI_n \cdot r(C_x, U \text{ or } G)_n$ where x has a value from 4 to 29, e.g., $rI_n \cdot r(C_{12}U)_n$ herein referred to as Ampligen®, a registered trademark of HEM Research, Inc., of Rockville, Maryland, USA. Many mismatched dsRNA polymers which behave similarly to Ampligen have been studied. The key therapeutic advantage of mismatched dsRNAs over other forms of natural and/or synthetic dsRNAs is their reduced toxicity in animals and man. For example, Lampson et al in U.S. Patent No. 3,666,646 described earlier complexes of dsRNA which are capable of triggering various interferon-related effects, but the toxicity of such compounds precluded any clinical utility in the treatment of cancer or related disorders.

4

By "mismatched dsRNA" are meant those in which hydrogen bonding (base stacking) between the counterpart strands is relatively intact, i.e., is interrupted on average less than one base pair in every 29 consecutive base residues. The term "mis-matched dsRNA" should be understood accordingly.

The dsRNA may be a complex of a polyinosinate and a polycytidylate containing a proportion of uracil bases or guanidine bases, e.g., from 1 in 5 to 1 in 30 such bases (poly I• poly (C4-29 x > U or G). The mismatched dsRNA may be poly I• poly C,U in which the ratio of C to U is about 13 to 1 and the sedimentation coefficients of poly I and poly C,U are both less than 9 and within 2 units of each other, that is preferably about 6.5 to 7.5.

The dsRNA may be of the general formula $rI_n.(C_{12-13}U)_n$. Other suitable examples of dsRNA are discussed below.

The mismatched dsRNAs preferred for use in the present invention are based on copolynucleotides selected from poly $(C_n,U)$ and poly $(C_n,G)$ in which n is an integer having a value of from 4 to 29 and are mismatched analogs of complexes of polyriboinosinic and polyribocytidilic acids, formed by modifying $rI_n.rC_n$ to incorporate unpaired bases (uracil or guanine) along the polyribocytidylate $(rC_n)$ strand. Alternatively, the dsRNA may be derived from poly (I). poly(C) dsRNA by modifying the ribosyl backbone of polyriboinosinic acid $(rI_n)$ e.g., by including 2'-O-methyl ribosyl residues. These mismatched analogs of $rI_n.rC_n$, preferred ones of which are of the general formula $rI_n.r(C_{12},U)_n$, are described by Carter and Ts'o in U.S. Patents 4,130,641 and 4,024,222 the disclosures of which are hereby incorporated by reference. The dsRNA's described therein generally are suitable for use according to the present invention.

Specific examples of mismatched dsRNA for use in the invention include: -

poly (I)• poly $(C_4,U)$
poly (I)• poly $(C_7, U)$
poly (I)• poly $(C_{13}, U)$
poly (I)• poly $(C_{22}, U)$
poly (I)• poly $(C_{20}, G)$
poly (I)• poly $(C_{29}, G)$ and
poly (I)• poly $(C_p)$ 23 G>p

Pharmaceutical compositions in accordance with this invention include the dsRNA, preferably mismatched, the lymphokine and, optionally a compound that inhibits cyclooxygenase activator, as the active components, together with a pharmaceutical carrier or diluent. Pharmaceutical compositions contemplated by this invention include those adapted for parenteral administration in a suitable pharmaceutical vehicle.

TABLE 1

| TOXICITIES REPORTED IN THE INITIAL LAK CELL/IL-2 CLINICAL TRIAL | |
|---|---|
| **Toxic Reaction** | **Percentage of Patients** |
| Malaise | 100 |
| Anemia requiring transfusion | 96 |
| Eosinophilia | 96 |
| Fever | 88 |
| Nausea or Vomiting | 84 |
| Dyspnea | 80 |
| Chills | 76 |
| Diarrhea | 72 |
| Erythema or Rash | 68 |
| Serum bilirubin > 2mg% | 64 |
| Weight gain | 64 |
| Pruritus | 64 |
| Glossitis | 56 |
| Nasal congestion | 52 |
| Serum creatinine > 2mg% | 48 |
| Thrombocytopenia | 44 |
| Confusion | 32 |
| Data from: Rosenberg, S.A., M.T. Lotze, L.M. Muul, et al. 1985. New Engl. J. Med. 313:1485. | |

TABLE 2

**CONCURRENT THERAPY GIVEN TO ALL PATIENTS DURING IL-2 ADMINISTRATION TO ABROGATE TOXICITY**

- **ACETAMINOPHEN** (fever and malaise)
- **INDOMETHACIN** (fever and malaise)
- **RANITIDINE** or **CIMETIDINE** (prophylaxis of gastrointestinal bleeding)
- **HYDROXYZINE HCl** or **DIPHENHYDRAMINE** (generalized erythematous rash)
- **MEPERIDINE** (concurrently with LAK cells to control chills and rigors)
- **ANTIEMETICS** and **ANTIDIARRHEAL** agents (as required)
- **DEXAMETHASONE** (control life threatening symptoms)

**Claims**

1. A product containing mismatched dsRNA and interleukin 2 as a combined preparation for simultaneous, separate or sequential use in the treatment of tumour, virus-infected or immune-disregulated cells, wherein the dsRNA is administered in an amount sufficient to result in a level of 0.1 to 1000 micrograms per millilitre of the patient's body fluid, and wherein the interleukin is administered in an

6

amount sufficient to result in the level of from $10^2$ to $10^6$ interleukin units per kilogram of the patient's body weight.

2. The product of claim 1, wherein the dsRNA is of the general formula $rI_n.(C_{12}U)n$.

**Patentansprüche**

1. Nicht richtig angepasstes doppelsträngiges RNS (dsRNS) und Interleukin 2 enthaltendes Produkt als kombiniertes Preparat zu einer gleichzeitigen, separaten oder sequentiellen Verwendung in der Behandlung von Tumorzellen, virusinfizierten Zellen oder immundefizienten Zellen, bei dem das dsRNS in einer ausreichenden Menge verabreicht wird, um einen Spiegel von 0.1 bis 1000 Mikrogramm pro Milliliter Körperflüssigkeit des Patienten zu erreichen, und bei dem das Interleukin in einer ausreichenden Menge verabreicht wird, um einen Spiegel von $10^2$ bis $10^6$ Einheiten Interleukin pro Kilogramm Körpergewicht des Patienten zu erreichen.

2. Produkt nach Anspruch 1, bei dem das dsRNS der Generalformel $rI_n.(C_{12}U)n$ entspricht.

**Revendications**

1. Produit contenant de l'ARN à double chaîne (dsARN) non-assorti et de l'interleukine 2 comme préparation combinée pour une utilisation simultanée, séparée ou séquentielle dans le traitement de cellules tumorales, de cellules infectées par un virus ou de cellules immuno-déficientes, par lequel l'ARN à double chaîne est administré en quantité suffisante pour atteindre un niveau allant de 0.1 à 1000 microgrammes par millilitre de liquide corporel du patient, et par lequel l'interleukine est administré en quantité suffisante pour atteindre de $10^2$ à $10^6$ unités d'interleukines par kilogramme de poids du corps du patient.

2. Produit de la revendication 1, dans lequel l'ARN à double chaîne correspond à la formule générale $rI_n.(C_{12}U)n$.

Fig. 1

# PRESENT THERAPY WITH rIL-2 AND LAK CELLS

IL2[a]        ↓↓↓↓↓↓↓↓↓↓↓↓↓↓↓↓        ↓↓↓[↓↓↓↓↓↓↓↓↓↓]

1        5        10        ↑↑    15↑

LAK[b]

Leukapheresis        ■ ■ ■ ■ ■

Treatment Days

[a]IL-2 infusion at 100,000 u/kg over 15 minutes every 8 hours
[b]LAK cell infusion:
    Day 12 - cells obtained from leukapheresis on days 8 and 9
    Day 13 - cells obtained from leukapheresis on day 10
    Day 15 - cells obtained from leukapheresis on days 11 and 12

Adapted from the NIH therapeutic protocol of LAK/IL-2 therapy reported
9/12/86 and Rosenberg, S.A., et al. New Engl J Med 313: 1485 (1985)

EP 0 281 380 B1

## TOXICITIES ASSOCIATED WITH INCREASING BOLUS DOSES OF IL-2 IN MAN

**Dose Limiting Toxicity**

- Hypotension
- Dyspnea
- Fluid Retention
- Ascites
- Anemia-Transfusions
- ↑ Creatinine
- ↑ Bilirubin
- Weight Gain
- Nausea/ Vomiting
- Fever/Chills
- Headache
- Malaise

Toxicity

$10^3$  $10^4$  $10^5$  $10^6$

**Approximate IL-2 Dose (u/kg)**

Data adapted from:
Lotze, M.T., et al. J. Immuriol. 134:157 (1985);
Lotze, M.T., et al. J. Immunol. 135:2865 (1985);
Rosenberg, S.A., et al. New Engl. J. Med..313:1485 (1985)
NIH Report 9/12/86

EP 0 281 380 B1

FIG.3

# AUGMENTATION OF HUMAN LAK KILLING
## BY $rI_n \cdot r(C_{12}, U)_n$

FIG. 4

INCREASED HUMAN LAK CELL ACTIVITY
BY THE COMBINATION OF rIL-2 (10 u/ml)
AND rI$_n$·r(C$_{12}$, U)$_n$ (200 µg/ml)

Fig. 5

# $r(I)_n \cdot r(C_{12},U)_n$/IL-2 COMBINATION TREATMENT REDUCES THE NUMBER OF MELANOMA LUNG METASTASES

p < 0.02

Average No. of Metastases per Lung

Control   IL-2   $r(I)_n \cdot r(C_{12},U)_n$   $r(I)_n \cdot r(C_{12},U)_n$ plus IL-2

FIG. 6

r(I)$_n$·r(C$_{12}$,U)$_n$/IL-2 THERAPY INCREASES SURVIVAL IN
MALIGNANT MELANOMA XENOGRAFT BEARING ATHYMIC MICE

● Control
▲ IL-2
○ r(I)$_n$·r(C$_{12}$,U)$_n$
△ r(I)$_n$·r(C$_{12}$,U)$_n$/IL-2

p=0.0325

% surviving fraction

Day

EP 0 281 380 B1

FIG. 7

# TOXICITIES ASSOCIATED WITH INCREASING BOLUS DOSES OF IL-2 IN MAN

**Projected IL-2 dose combined with dsRNA**

**Current IL-2 dose**

Dose Limiting Toxicity

- Hypotension
- Dyspnea
- Fluid Retention
- Ascites
- Anemia-Transfusions
- ↑Creatinine
- ↑Bilirubin
- Weight Gain
- Nausea/Vomiting
- Fever/Chills
- Headache
- Malaise

Toxicity

$10^3$     $10^4$     $10^5$     $10^6$

## Approximate IL-2 Dose (u/kg)

Data adapted from:
Lotze, M.T., et al. J. Immunol. 134:157 (1985);
Lotze, M.T., et al. J. Immunol. 135:2865 (1985);
Rosenberg, S.A., et al. New En' J. Med. 313:1485 (1985)
NIH Report 9/12/86

EP 0 281 380 B1